# EUROPÄISCHE PATENTSCHRIFT

(11) **EP 3 035 877 B1**
(45) Veröffentlichungstag und Bekanntmachung des Hinweises auf die Patenterteilung: **01.07.2020**
(21) Anmeldenummer: 14755060.2
(22) Anmeldetag: 20.08.2014
(51) Int. Cl.: A61B 17/70

(54) **CHIRURGISCHES INSTRUMENT ZUM MANIPULIEREN, POSITIONIEREN UND FIXIEREN EINES CHIRURGISCHEN STABES RELATIV ZU EINEM IMPLANTAT**
SURGICAL INSTRUMENT FOR MANIPULATING, POSITIONING AND FIXING A SURGICAL ROD IN RELATION TO AN IMPLANT
INSTRUMENT CHIRURGICAL PERMETTANT DE MANIPULER, POSITIONNER ET FIXER UNE TIGE CHIRURGICALE PAR RAPPORT À UN IMPLANT CHIRURGICAL

(30) Priorität: 21.08.2013 DE 102013109058
(43) Veröffentlichungstag der Anmeldung: 29.06.2016
(73) Patentinhaber: Aesculap AG, 78532 Tuttlingen (DE)
(72) Erfinder: FISCHER, Kay, 78532 Tuttlingen (DE); KRUEGER, Sven, 78647 Trossingen (DE); MARX, Irene, 78647 Trossingen (DE); SCHNEIDER, Lisa, 72275 Alpirsbach (DE)
(74) Vertreter: Winter, Brandl, Fürniss, Hübner, Röss, Kaiser, Polte - Partnerschaft mbB
(86) Internationale Anmeldenummer: PCT/EP2014/067760
(87) Internationale Veröffentlichungsnummer: WO 2015/024976

(56) Entgegenhaltungen:
- US-A- 6 036 692
- US-A1- 2008 234 765
- US-A1- 2010 228 302
- US-A1- 2011 184 469

## Beschreibung

Die vorliegende Erfindung betrifft ein chirurgisches Instrument, mit dem ein chirurgischer Stab relativ zu einem Implantat, insbesondere zu einer Pedikelschraube, manipuliert, positioniert und fixiert werden kann.

Die Verwendung von Pedikelschrauben ist in der Chirurgie allgemein bekannt. Sie werden in der Regel zusammen mit einem Stab verwendet, um beispielsweise Wirbelkörper zueinander zu fixieren. Dabei werden die Pedikelschrauben in zueinander zu fixierende Wirbelkörper geschraubt und nachfolgend üblicherweise mittels eines Stabs miteinander verbunden. Zu diesem Zweck weist der Kopf der Pedikelschraube, der auch als Tulpe bezeichnet wird, eine meist U-förmige Aussparung auf. Zum Fixieren von Knochen oder Knochenteilen zueinander wird der Stab in die Tulpe eingelegt und dort mittels einer Verschraubung geklemmt, so dass die Pedikelschrauben und damit auch die Knochen oder Knochenteile, in die diese eingeschraubt sind, über den Stab zueinander fixiert sind. Das Klemmen des Stabs erfolgt in der Regel mit Madenschrauben, sogenannten Setscrews.

Unter Umständen kann es vorkommen, dass der Stab vor dem Klemmen nicht oder nur teilweise in der Tulpe liegt. In einem solchen Fall muss er zunächst korrekt in der Tulpe positioniert werden und kann erst nachfolgend in dieser Position mittels Einschrauben der Setscrew in der Tulpe geklemmt werden. Um den Stab in der Ausnehmung der Pedikelschraube zu positionieren, wird er mit einem Rod Pusher oder Rod Rocker genannten Instrument in die Tulpe hinein gedrückt oder die Pedikelschraube an ihn herangezogen.

Der mittels eines solchen Instruments in die Tulpe gezwungene Stab steht zwangsläufig meist unter Spannung. Dann ist es notwendig, die Setscrew zumindest teilweise in die Tulpe einzuschrauben, während der Stab mittels des Rod Pushers gehalten wird, so dass er in der dazu bestimmten Ausnehmung der Tulpe verbleibt. Das Einschrauben der Setscrew erfolgt in der Regel mit einem Schraubendreher, wobei dafür Sorge zu tragen ist, dass sich die Pedikelschraube beim Einschrauben der Setscrew nicht verdreht. Abhängig vom Design des Implantates ist es von Vorteil oder sogar zwingend notwendig, die Setscrew genau in Richtung der Gewindeachse einzubringen, was bei bestimmten Operationsbedingungen problematisch sein kann. Aus dem Stand der Technik ist zu diesem Zweck bekannt, zur Führung der Setscrew und/oder des Schraubendrehers eine Führungsgeometrie im Implantat vorzusehen. Es ist auch bekannt, neben dem Rod Pusher ein zusätzliches Führungsinstrument einzusetzen. Um ein Verdrehen der Pedikelschraube beim Eindrehen der Setscrew zu vermeiden, ist die Verwendung von Gegenhaltern bekannt, mit denen die Pedikelschraube manuell durch den Chirurgen oder anderes OP-Personal gehalten wird.

Zum Positionieren eines chirurgischen Stabs in einem Implantat sind aus dem Stand der Technik verschiedene Instrumente bekannt. Ein Beispiel ist ein hebelartiges Instrument, dessen eine Seite einen Handgriff und dessen gegenüberliegende Seite einen gabelartigen Instrumentenkopf aufweist. Der Instrumentenkopf ist schwenkbar am Kopf eines Implantats, z.B. einer Pedikelschraube, anordbar und besitzt eine hebelartig beabstandete Anlagefläche für den chirurgischen Stab. Durch Schwenken des Instruments um den Kopf des Implantats wird die Anlagefläche für den chirurgischen Stab in Richtung der Stabaufnahme der Pedikelschraube bewegt. Der Stab kann dann mittels des Instruments in der gewünschten Position gehalten werden. Nachteilig ist, dass die Anordnung und Positionierung des Instruments am Implantat mit einer gewissen Unsicherheit behaftet ist, so dass es sich unter Umständen lösen kann. Des Weiteren erfüllt das Instrument ausschließlich die Funktion der Positionierung und des Haltens des Stabs in der Stabaufnahme der Pedikelschraube und muss zu diesem Zweck manuell in der dazu erforderlichen Position gehalten werden. Es bietet mit Nachteil keine Führung, die dem Chirurgen beim Einschrauben der Setscrew behilflich ist.

Ein anderes Beispiel für ein bekanntes Instrument ist eine Zange, deren implantatseitigen Zangenarme zu einer Aufnahme für den Kopf einer Pedikelschraube ausgebildet sind. Die Zange kann durch den Chirurgen geöffnet, den Pedikelschraubenkopf umfassend an dieser angeordnet und nachfolgend geschlossen werden, so dass die Aufnahme der Zange sicher am Schraubenkopf angeordnet ist. Die Zange weist eine Ratsche auf, mittels der sie in der geschlossenen Stellung verbleibt und ohne Lösen der Ratsche nicht von der Pedikelschraube entfernt werden kann. Die Zangenarme sind hebelartig ausgebildet und weisen im geschlossenen Zustand zwischen sich eine Anlagefläche für den chirurgischen Stab auf, so dass dieser durch Schwenken der Zange um den Pedikelschraubenkopf in die dazu vorgesehene Aufnahme der Tulpe eingebracht werden kann. Die Zange kann einfach am Pedikelschraubenkopf angeordnet werden, allerdings sind für einen dauerhaft sicheren Sitz am Pedikelschraubenkopf eine korrekte Positionierung und die Aufbringung eines ausreichenden Schließdrucks erforderlich. Auch muss auch die Zange manuell in der zum Positionieren und Klemmen des chirurgischen Stabs erforderlichen Position gehalten werden. Das Instrument bietet mit Nachteil keine Führung, die dem Chirurgen beim Einschrauben der Setscrew behilflich ist.

Eine andere bekannte Vorrichtung besitzt axial ausgerichtete, zueinander konzentrische Hülsen, die mittels eines Hebelratschenmechanismus in axialer Richtung zueinander bewegbar sind. Eine innere Hülse wird am Kopf einer Pedikelschraube angeordnet. Eine zu der inneren Hülse bewegbare äußere Hülse besitzt eine Aufnahme für einen chirurgischen Stab oder bildet eine solche Aufnahme aus. Durch eine Betätigung des Hebelmechanismus bewegt sich die äußere Hülse zusammen mit dem chirurgischen Stab auf die innere Hülse, die am Pedikelschraubenkopf angeordnet ist, zu, so dass der Stab relativ zum Schraubenkopf in dessen Aufnahme positioniert wird. Die Innenräume der inneren und der äußeren Hülse bilden gleichzeitig eine zum Pedikelschraubenkopf stets ausgerichtete Führung für ein Schraubwerkzeug zum Einschrauben einer Setscrew aus.

Aus der Patentschrift US 8,449,549 B2 ist schließlich ein als Rod Coercer bezeichnetes zangenartiges Instrument in mehreren Ausführungsformen bekannt. Es umfasst zwei drehbar um ein erstes Scharnier aneinander angeordnete Zangenbranchen mit Griffen für eine nutzerseitige Betätigung. Die der Griffseite gegenüberliegenden pedikelschraubenseitigen Enden der Zangenbranchen bilden gemeinsam im geschlossenen Zustand der Zange eine Anlagefläche für einen chirurgischen Stab aus. An den pedikelschraubenseitigen Enden ist um ein zweites Scharnier, dessen Schwenkachse zu der des ersten Scharniers orthogonal ist, schwenkbar jeweils ein Implantatgreifarm angeordnet. Bei einem Öffnen des Instruments öffnen sich auch dessen Implantatgreifarme sowie dessen pedikelschraubenseitige Enden. Bei einem Schließen des Instruments schließen sich dessen Implantatgreifarme sowie dessen pedikelschraubenseitigen Enden. Das Instrument wird im geöffneten Zustand an einer Pedikelschraube angesetzt und nachfolgend geschlossen. Dabei sollen die Implantatgreifarme den Pedikelschraubenkopf umschließen und die pedikelschraubenseitigen Enden mehr oder weniger definiert aneinander anliegen und die Anlagefläche für den chirurgischen Stab ausbilden. Die pedikelschraubenseitigen Enden sind aufgrund der zwei zueinander orthogonalen Schwenkachsen der Scharniere in zwei Raumrichtungen zueinander frei beweglich. In der Folge kann bei einem Gebrauch des Instruments das rechte Arbeitsende eine andere Position als das linke Arbeitsende aufweisen, und ein Verkippen der beiden Arbeitsenden zueinander ist möglich. Das Instrument ermöglicht dem Chirurgen in der Folge kein direktes Gefühl und Feedback hinsichtlich der Situation am Pedikelschraubenkopf. Die Handhabung des Instruments und insbesondere dessen Positionierung relativ zum Pedikelschraubenkopf und dem chirurgischen Stab sind schwierig. Das Instrument besitzt keine Führungseinrichtung für die Setscrew oder für ein Werkzeug zum Einschrauben der Setscrew. Die Funktion des Instruments als Gegenhalter, um beim Einschrauben der Setscrew ein Verdrehen der Pedikelschraube zu verhindern, hängt mit Nachteil von der korrekten Positionierung auf dem Pedikelschraubenkopf und einem ausreichend hohen Schließdruck des Instruments ab.

Weitere gattungsgemäße chirurgische Instrumente sind aus den Druckschriften US 2011/0184469 A1, US 6,036,692 A, US 2008/0234765 A1 und US 2010/0228302 A1 bekannt.

Ausgehend vom vorstehend beschriebenen Stand der Technik liegt der Erfindung die Aufgabe zugrunde, einem Chirurgen das Manipulieren, Positionieren und Fixieren eines chirurgischen Stabs relativ zu einem in einem Knochen oder einem Knochenstück eingeschraubten Implantat, insbesondere einer Pedikelschraube, in verlässlicher und schneller Weise zu ermöglichen. Dabei soll insbesondere das Einschrauben einer Fixierungsschraube für den Stab in das Implantat erleichtert werden. Das Instrument soll einfach und zu günstigen Kosten herzustellen und für den nutzenden Chirurgen einfach anzuwenden sein.

Die Aufgabe wird gelöst durch ein chirurgisches Instrument zum Manipulieren und Positionieren eines chirurgischen Stabs relativ zu einem chirurgischen Implantat, insbesondere einer Pedikelschraube, gemäß den Merkmalen des Anspruchs 1.

Das Instrument ist zangenartig mit zwei Branchen ausgebildet, die über ein Scharnier drehbar miteinander gekoppelt sind, wobei jede der Branchen ein Arbeitsende aufweist, welche Arbeitsenden bestimmt und eingerichtet sind, beim Schließen des Instruments durch Drehen der Branchen um das Scharnier mit dem Implantat in Eingriff zu gelangen und das Instrument relativ zum Implantat um eine Schwenkachse quer zu dessen Längsachse verschwenkbar zu positionieren, wobei zumindest eine der Branchen eine Anlagestruktur für einen chirurgischen Stab aufweist, mit der der Stab bei einem Verschwenken des Instruments um die Schwenkachse relativ zu einer Stabaufnahme des Implantats manipuliert und positioniert wird, und wobei an zumindest einer der Branchen eine Führungsstruktur ausgebildet ist, zum Führen einer nach der Positionierung des Stabs in der Stabaufnahme einzuschraubenden Fixierungsschraube für den Stab und/oder eines Werkzeugs zum Einschrauben der Fixierungsschraube.

Die vorliegende Erfindung ermöglicht, die Funktionen des Manipulierens und Positionierens des Stabs einerseits zusammen mit einer Führung der Fixierungsschraube und/oder des Werkzeugs zum Einschrauben der Fixierungsschraube andererseits kombiniert mittels Verwendung eines einzigen Instruments zu verwirklichen und erspart dem Operateur das Aufsetzen und Halten eines separaten Führungsinstrumentes sowie die Notwendigkeit einer dritten Hand bzw. eines Assistenten. Die Handhabung des Instruments ist daher deutlich vereinfacht gegenüber dem Stand der Technik. Da ein separates Handhaben eines Führungsinstruments für die Fixierungsschraube bzw. das Werkzeug zum Eindrehen der Fixierungsschraube nicht notwendig ist, kann bei einer OP mit Vorteil Zeit eingespart werden. Das Instrument nach der Erfindung ist mit Vorteil komplett einhändig zu bedienen, so dass der Chirurg seine freie Hand für andere Zwecke, insbesondere zum Einschrauben der Fixierungsschraube, nutzen kann. Das Einschrauben der Fixierungsschraube wird deutlich vereinfacht und Fehler werden vermieden. Dies ist insbesondere bei unübersichtlichen OP-Bedingungen von Vorteil, wenn der Chirurg den Stab ohne ausreichende Sicht positionieren und fixieren muss.

Nach dem Niederdrücken des Stabs kann die Fixierungsschraube im Wesentlichen kräftefrei und seitlich geführt in die Stabaufnahme bzw. Tulpe eindreht werden. Dadurch wird verhindert, dass die Fixierungsschraube schief in das Gewinde gedreht (Cross-Threading) und unter Umständen das Gewinde dabei beschädigt wird.

Nach einer Ausführungsform kann wenigstens ein Arbeitsende der Branchen des Instruments einen Zapfen oder eine ähnliche Struktur aufweist, der bzw. die beim Schließen des Instruments durch Drehen der Branchen um das Scharnier in eine dazu vorgesehene Ausnehmung oder ähnliche Struktur in dem Implantat, insbesondere im Kopf des Implantats bzw. einer Tulpe einer Pedikelschraube, eingreift und darin relativ zum Implantat um die Achse des Zapfens drehbar gehalten ist. Bei dieser Ausführungsform ist in vorteilhafter Weise das gesamte Instrument um die durch den Zapfen ausgebildete Drehachse schwenkbar. Dem Chirurgen wird daher ein deutliches Gefühl vermittelt, ob z. B. das Instrument korrekt am Implantat angeordnet ist und ob der Stab korrekt und in einer gewünschten Lage in dem Implantat positioniert ist. Auch ist ein sicherer Sitz des Instruments am Implantat sichergestellt. Durch die Anordnung von Zapfen in Ausnehmungen des Implantats ist das Instrument gegenüber dem Implantat zwar um die durch die Zapfen und Ausnehmungen gebildete Schwenkachse quer zur Implantatachse schwenkbar, allerdings nicht um die Implantatachse. Auf diese Weise wirkt das Instrument mit Vorteil als Verdrehsicherung beim Einschrauben der Fixierungsschraube. Nach dem Stand der Technik war zu diesem Zweck ein separater Gegenhalter erforderlich, so dass durch diese Ausführungsform der Erfindung eine weitere Vereinfachung für den Chirurgen erreicht wird. Des Weiteren kann die Fixierungsschraube mit einem hohem Drehmoment angezogen werden, ohne dass dieses auf das Implantat übertragen wird.

Nach einer weiteren Ausführungsform ist die Führungsstruktur in Form einer vorzugsweise ebenen Führungsfläche ausgebildet, die orthogonal zur Schwenkachse des Instruments orientiert ist und unabhängig vom Schwenkwinkel des Instruments relativ zum Implantat die Führung für die Fixierungsschraube und/oder das Werkzeug zum Einschrauben der Fixierungsschraube bildet.

Mit anderen Worten die Führungsstruktur ist derart ausgebildet, dass sie über den gesamten zu erwartenden Schwenkbereich des Instruments eine Führung oder seitliche Abstützung der Fixierungsschraube und/oder das Werkzeug bietet bzw. dass sich die Führungsstruktur dem jeweiligen Schwenkwinkel folgt.

Nach einer weiteren Ausführungsform ist die Anlagestruktur an einer der Branchen ausgebildet und erstreckt sich bei geschlossenem Instrument im Wesentlichen quer über den gesamten Bereich zwischen den Branchen. So ist auch bei einem nicht vollständig geschlossenen Instrument eine sichere Anlage des Stabs gewährleistet.

Nach einer Ausführungsform ist an dem Arbeitsende jeder Branche jeweils ein bogenförmiger Vorsprung einstückig angeformt, wobei die Innenflächen der beiden Vorsprünge im Wesentlichen eine Verlängerung der Stabaufnahme bilden und eine seitliche Führung für die Fixierungsschraube und/oder das Werkzeug zum Einschrauben der Fixierungsschraube bilden.

Nach einer Ausführungsform sind die Innenflächen der Vorsprünge in der geschlossenen Stellung des Instruments planparallel zur Schwenkebene.

Nach einer Ausführungsform entspricht die Bogenlänge des Vorsprungs dem zu erwartenden Schwenkwinkel des Instruments. Somit wird sichergestellt, dass die Fixierungsschraube und/oder das Werkzeug zum Einschrauben der Fixierungsschraube während der gesamten Schwenkbewegung seitlich geführt sind.

Nach einer weiteren Ausführungsform weist wenigstens eine der Branchen eine auf einem Kreisbogen um die Schwenkachse des Instruments relativ zum Implantat geführte Führungseinheit mit einer teilzylinderförmigen oder zylinderförmigen Führungsfläche für die Fixierungsschraube und/oder das Werkzeug zum Einschrauben der Fixierungsschraube auf. Bei dieser Ausführungsform ist eine Führung der Fixierungsschraube bzw. des Einschraubwerkzeugs in zwei Raumrichtungen orthogonal zur Längsachse des Implantats stets gewährleistet.

Nach einer weiteren Ausführungsform weist die Führungseinheit einen Bolzen auf, der in einer in der Branche ausgebildeten bogenförmigen Nut oder Führungsbahn geführt ist. Der Bolzen kann einen runden, ovalen oder langgestreckten Querschnitt aufweisen. Bei einem Bolzen mit rundem Querschnitt ist der Bolzen in der Führungsnut und damit die gesamte Führungseinheit frei drehbar, was unter gewissen Umständen, z.B. bei einem schiefen oder verkanteten Ansetzen des Instruments am Implantat eine korrekte Anordnung und Anlage der Führungseinheit am Implantat erleichtert. Bei anderen Querschnittsformen wird diese freie Drehbarkeit verhindert und eine Ausrichtung der Führungseinheit relativ zur Schwenkachse des Instruments um das Implantat ist stets sichergestellt.

Insbesondere kann der Bolzen in der Nut mittels einer Feder insbesondere als Rückstellglied in eine Endlage vorgespannt sein. In dieser Endlage ist die Führungseinheit mit Vorteil derart zum Instrument angeordnet und ausgerichtet, dass sie bei einem Ansetzen des Instruments am Implantat flach auf dem Implantat aufliegt, wobei die Führungsflächen der Führungseinheit bereits in einer geeigneten Orientierung zum Führen der Fixierungsschraube bzw. des Werkzeugs ausgerichtet sind. Nachdem das Instrument von dem Implantat gelöst wurde, stellt das Rückstellglied die Führungseinrichtung zurück in die Ausgangsposition.

Nach einer weiteren Ausführungsform kann die Führungseinheit eine zylinderförmige Durchgangsöffnung aufweisen, deren Innenwand die Führungsfläche für die Fixierungsschraube und/oder das Werkzeug zum Einschrauben der Fixierungsschraube ausbildet. Bei dieser Ausführungsform ist eine Führung der Fixierungsschraube bzw. des Einschraubwerkzeugs in zwei Raumrichtungen orthogonal zur Längsachse des Implantats stets gewährleistet. Ein Abrutschen des Werkzeugs ist durch die geschlossene Bauweise der Führung nicht zu erwarten und kann sicher verhindert werden. Nach einer weiteren Ausführungsform kann die Führungseinheit zweigeteilt sein und an jeder der Branchen ein Teil der Führungseinheit angeordnet sein. Auf diese Weise kann das zweite Arbeitsende ebenfalls eine Zwangsführung, ein Rückstellglied und eine Führungseinrichtung für einen Lagerungszapfen bzw. eine gegen Verdrehen gesicherte Lagerung aufweisen.

Weitere Merkmale und Vorteile der vorliegenden Erfindung ergeben sich aus der folgenden beispielhaften Beschreibung besonders bevorzugter Ausführungsformen der Erfindung anhand der Figuren. Dabei zeigt:
Fig. 1 in einer schematischen perspektivischen Darstellung einen mit zwei Pedikelschrauben zu verbindenden Stab,
Fig. 2 in einer schematischen perspektivischen Darstellung eine erste Ausführungsform des Instruments,
Fig. 3 einen Arbeitsbereich einer Branche des Instruments der Figur 2 in einer vergrößerten schematischen perspektivischen Darstellung,
Fig. 4 das Aufsetzen des Instruments der Figuren 2 und 3 auf eine Pedikelschraube als ersten Arbeitsgang aus zwei unterschiedlichen Blickrichtungen,
Fig. 5 das Herunterdrücken des Stabes als zweiten Arbeitsgang aus zwei unterschiedlichen Blickrichtungen,
Fig. 6 das Einschrauben einer Fixierungsschraube in die Pedikelschraube,
Fig. 7 eine zweite Ausführungsform des Instruments in einer schematischen perspektivischen Darstellung,
Fig. 8 die Verwendung der Ausführungsform der Figur 7 in zwei schematischen Ansichten und
Fig. 9 eine dritte Ausführungsform des Instruments in einer schematischen perspektivischen Darstellung.

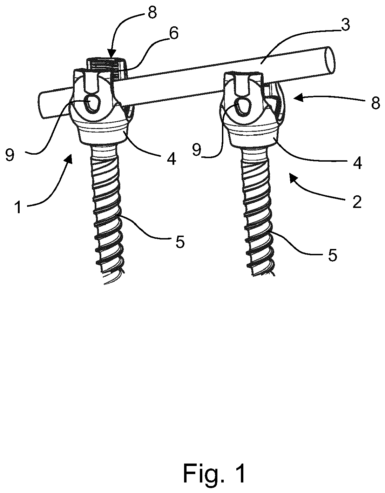

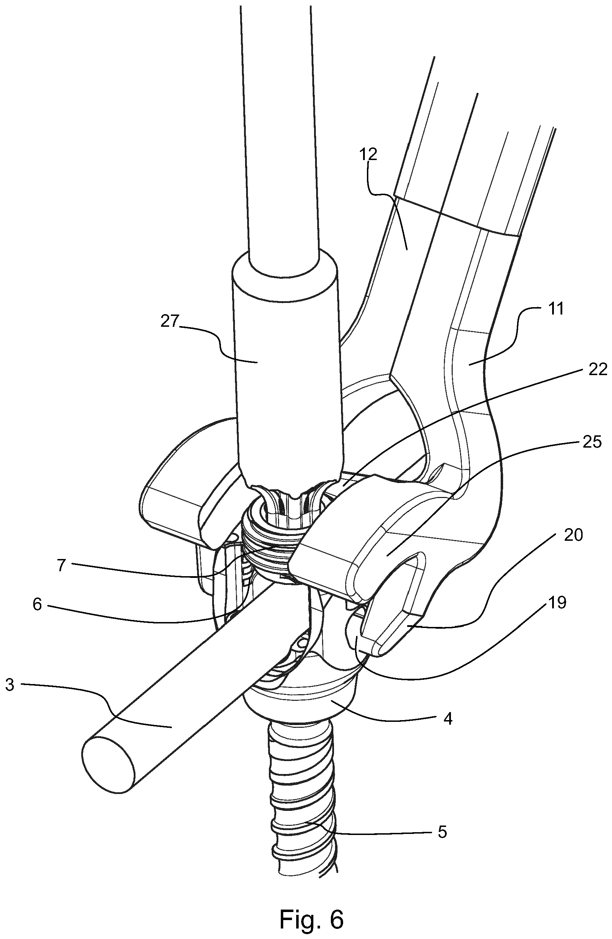

Die Figur 1 zeigt schematisch eine Situation während eines spinalen Eingriffs, bei dem zwei in Figur 1 nicht dargestellte Wirbel mittels zweier Pedikelschrauben 1 und 2 als Implantate und eines chirurgischen Stabs 3 zueinander fixiert werden. Jede Pedikelschraube 1, 2 weist einen Kopf 4 auf, der auch als Tulpe bezeichnet wird. Der Kopf 4 ist im Wesentlichen U-förmig ausgebildet und weist eine Ausnehmung oder Stabaufnahme 8 auf, die zur Klemmung des Stabs 3 eingerichtet und bestimmt ist. An den Kopf 4 anschließend besitzt die Pedikelschraube 1, 2 einen Schaft 5, der mit einem Knochengewinde versehen ist, das in den Wirbel eingeschraubt wird. In der Stabaufnahme 8 des Kopfs 4 ist ein Innengewinde 6 ausgebildet, in das eine in der Figur 1 nicht dargestellte Fixierungsschraube 7 (siehe z.B. in Figur 6) für den Stab 3, die auch als Setscrew bezeichnet wird, einzuschrauben ist. Der Stab 3 ist in der in Figur 1 dargestellten Situation in die Stabaufnahme 8 der Pedikelschraube 1 eingelegt, jedoch noch nicht fixiert. An der von der Stabaufnahme 8 abgewandten Außenseite des Kopfs 4 sind zwei einander gegenüberliegende Ausnehmungen 9 ausgebildet, von denen in Figur 1 jeweils nur eine sichtbar ist. Diese dienen, wie nachfolgend genauer ausgeführt wird, als Ansätze für das Instrument an der Pedikelschraube.

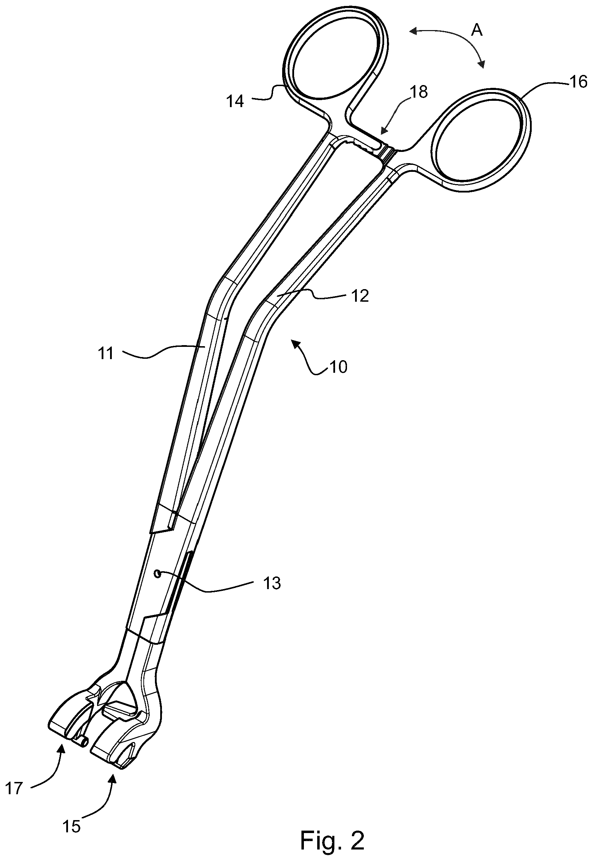

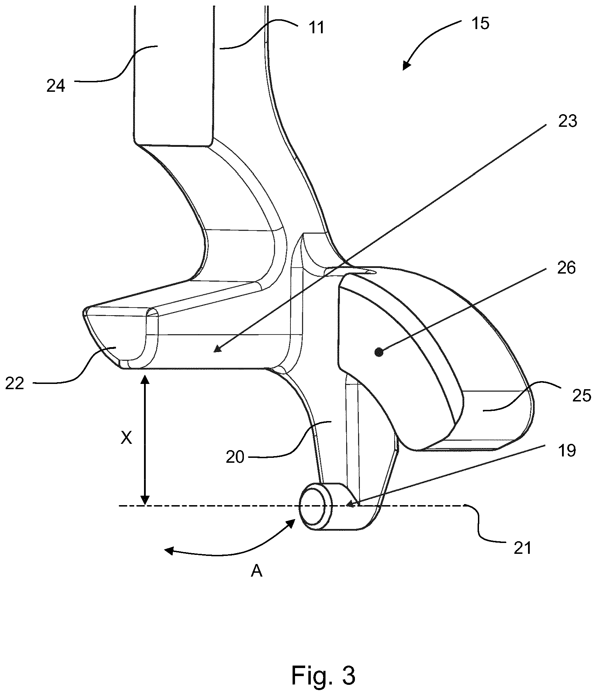

Eine erste Ausführungsform des Instruments 10 ist in Figur 2 gezeigt. Das Instrument 10 ist zangenartig mit zwei Branchen 11 und 12 ausgebildet und in Figur 2 im geschlossenen Zustand gezeigt. Die beiden Branchen 11 und 12 sind mittels eines Scharniers 13 relativ zueinander um dessen Achse drehbar gekoppelt. Die Branche 11 weist endseitig eine Fingeröse 14 und ein am gegenüberliegenden Ende ausgebildetes Arbeitsende 15 auf, das im Detail in Figur 3 dargestellt ist. Die Branche 12 weist endseitig eine Fingeröse 16 und ein am gegenüberliegenden Ende ausgebildetes Arbeitsende 17 auf. Im Bereich der Fingerösen 14, 16 sind die Branchen 11, 12 mittels eines Ratschenmechanismus 18 miteinander verrastet bzw. verrastbar. Unter Lösen des Ratschenmechanismus 18 können die Branchen 11, 12 auseinandergespreizt werden, wobei sich die beiden Arbeitsenden 15, 17 voneinander entfernen.

Die Bewegungsrichtung des Arbeitsendes 15 beim Öffnen und Schließen des Instruments 10 ist in Figur 3 mittels eines Doppelpfeils A gekennzeichnet. Das Arbeitsende 15 weist einen Zapfen 19 auf, dessen Zapfenachse 21 sich im Wesentlichen in die Bewegungsrichtung erstreckt, also orthogonal zur Achse des Scharniers 13 ist. Der Zapfen 19 ist am Ende eines Zapfenarms 20 angeordnet, ragt in Richtung des (nicht dargestellten) Arbeitsendes 17 der anderen Branche 12 vor und ist dazu eingerichtet und bestimmt, in eine Ausnehmung 9 der Pedikelschraube 1, 2 einzugreifen, wenn das Instrument 10 an dieser angeordnet und geschlossen wird.

Von der Zapfenachse 21 um einen Hebelarm X beabstandet ist am Arbeitsende 15 ein in Richtung des gegenüberliegenden Arbeitsendes 17 ragender Vorsprung 22 als Anlagestruktur für einen chirurgischen Stab 3 ausgebildet. Der Vorsprung 22 weist eine bogenförmige Anlagefläche 23 auf, an der der Stab 3 bei einer Manipulation mittels des Instruments 10 anliegt und abrollt oder gleitet. Der Vorsprung 22 ragt über die Teilungsebene des Instruments 10, in der die in Figur 3 mit der Bezugsziffer 24 bezeichnete Innenfläche der Branche 11 verläuft, hinaus und erstreckt sich des Instruments 10 im Wesentlichen quer über den gesamten sich im geschlossenen Zustand zwischen den Arbeitsenden 15,17 erstreckenden Bereich zwischen diesen. Die Zapfenachse 21 ist im Wesentlichen parallel zur Anlagefläche 23.

Das Arbeitsende 15 weist schließlich einen Vorsprung 25 auf, der tangential zur Zapfenachse 21 ausgerichtet ist. Der Vorsprung 25 ist bogenförmig ausgebildet und von der Zapfenachse 21 um seinen Krümmungsradius R beabstandet. An der zum gegenüberliegenden Arbeitsende 17 der Branche 12 weisenden Seite des Vorsprungs 25 ist eine ebene Führungsfläche 26 ausgebildet. Diese ist für eine Führung einer Setscrew 7 und/oder eines Werkzeugs 27 zu deren Einschrauben bestimmt und eingerichtet.

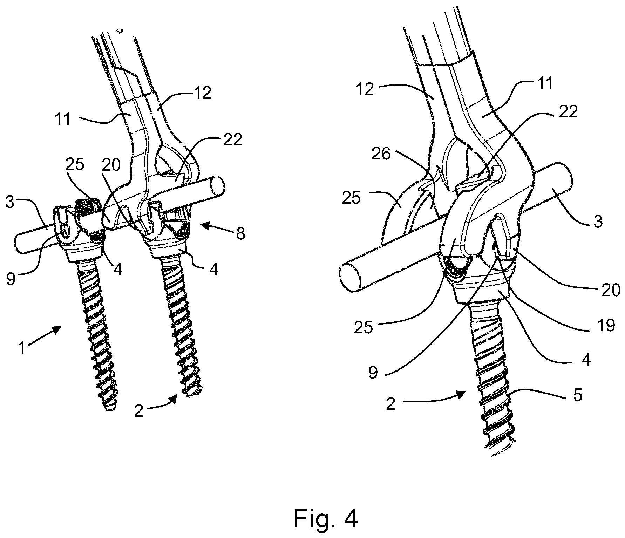

Das Arbeitsende 17 der Branche 12 ist im Wesentlichen spiegelverkehrt zum Arbeitsende 15 ausgebildet. Allerdings weist das Arbeitsende 17 keinen Vorsprung 22 als Anlagestruktur für den chirurgischen Stab 3 auf (gut erkennbar in Figur 4). Im geschlossenen Zustand des Instruments 10 weisen die gegenüberliegenden Führungsflächen 26 der Vorsprünge 25 einen ausreichenden Abstand voneinander auf, so dass der Stab 3 mit Spiel zwischen ihnen angeordnet sein kann. Der Abstand zwischen den Führungsflächen 26 entspricht in etwa dem Durchmesser einer Setscrew oder eines Werkzeugs, um diese einzuschrauben, so dass eine Führung in einer Raumrichtung (quer zur Scharnierachse des Scharniers 13) erzielt wird.

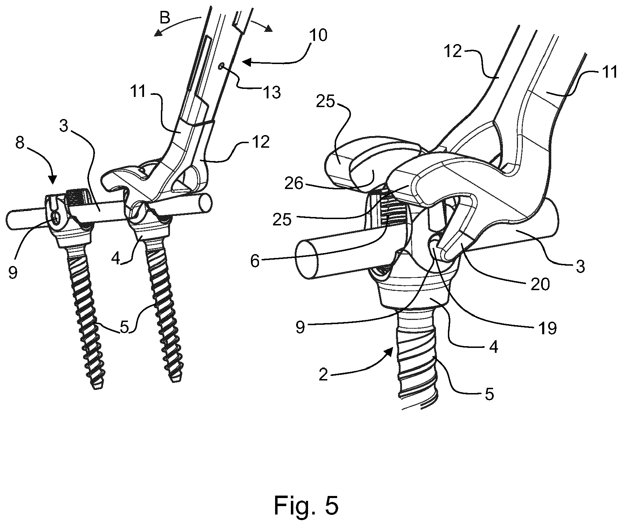

Die Verwendung und Funktion der in den Figuren 2 und 3 gezeigten Ausführungsform wird nun mit Bezug auf die Figuren 4 bis 6 beschrieben. In Figur 4 ist das Instrument 10 nach Ansetzen an die Pedikelschraube 2 aus zwei verschiedenen Blickrichtungen gezeigt. Um das Instrument 10 anzusetzen, wird es von einer Bedienperson an den Fingerösen 14 und 16 ergriffen und geöffnet, so dass sich die Arbeitsenden 15 und 17 und damit die Zapfen 19 voneinander entfernen. Im geöffneten Zustand kann das Instrument 10 an dem Kopf 4 der Pedikelschraube 2 angeordnet und geschlossen werden. Beim Schließen des Instruments 10 dringen die Zapfen 19 der beiden Arbeitsenden 15 und 17 in die zu diesem Zweck am Kopf 4 vorgesehenen Ausnehmungen 9 ein. Diese bilden zusammen mit den darin eingreifenden Zapfen 19 ein Schwenklager aus, dessen Schwenkachse quer zur Längsachse der Pedikelschraube 1, 2 verläuft und um das das gesamte Instrument 10 relativ zur Pedikelschraube 2 und zum Stab 3 schwenkbar ist. Die entsprechende Schwenkrichtung ist in Figur 5 mittels eines Doppelpfeils B verdeutlicht. Beim Schließen des Instruments 10 (Bewegungsrichtung A) gelangt der Ratschenmechanismus 18 in Eingriff, hält die Branchen 11, 12 des Instruments 10 in der entsprechend geschlossenen Position zueinander und sperrt es gegen unbeabsichtigtes Öffnen. Das Instrument 10 ist in dem in Figur 4 gezeigten Zustand also über den Ratschenmechanismus 18 verriegelt und verbleibt ohne bewusste nutzerseitige Betätigung in dieser Position. Wie insbesondere die linke Abbildung der Figur 4 zeigt, ist der Stab 3 über der Stabaufnahme 8 positioniert und noch nicht in diese eingeführt.

Um dem Stab 3 in die Stabaufnahme 8 zu drücken, wird das Instrument 10 im weiterhin geschlossenen Zustand durch den Chirurgen um die durch die Zapfen 19 und die Ausnehmungen 9 gebildete Schwenkachse, also die Zapfenachse 21, geschwenkt. Diese Bewegung ist schematisch in zwei Ansichten aus unterschiedlichen Blickrichtungen in Figur 5 gezeigt. Bei der genannten Schwenkbewegung um die Zapfenachse gelangt der Vorsprung 22 mit seiner bogenförmigen Anlagefläche 23 mit dem Stab 3 in Eingriff und drängt diesen bei fortgeführter Schwenkbewegung weiter in die Stabaufnahme 8. Die gegenüberliegenden Vorsprünge 25 bewegen sich bei dieser Schwenkbewegung auf einer entsprechenden bogenförmigen Bahn um die Ausnehmungen 9 der Pedikelschraube, wobei die Führungsflächen 26 unabhängig vom jeweiligen Schwenkwinkel stets kurz oberhalb des Kopfes 4 mit der Stabaufnahme 8 bleiben. Auf diese Weise ist eine Führung der Setscrew bzw. des Einschraubwerkzeugs für diese unabhängig vom Schwenkwinkel, der von der Eindrücktiefe des Stabs 3 in die Stabaufnahme 8 abhängt, sichergestellt.

Figur 6 zeigt schließlich das Einschrauben der Setscrew 7 mittels eines Werkzeugs 27 in das Innengewinde 6 des Kopfs 4 der Pedikelschraube 1, 2. Es ist zu erkennen, dass die einander gegenüberliegenden Führungsflächen 26 zunächst die Setscrew 7 und bei fortgesetztem Einschrauben das Werkzeug 27 seitlich führen. Bei der dargestellten Ausführungsform erfolgt die Führung quer zum Stab 3. Aus Figur 6 geht des Weiteren hervor, dass das Instrument 10 neben der beschriebenen Funktion der Manipulation und Positionierung des Stabs 3 relativ zur Pedikelschraube 1, 2 und des Einführens des Stabs 3 in die Stabaufnahme 8 als weitere Funktion die eines Gegenhalters übernimmt, der bei einem Einschrauben der Setscrew 7 in das Innengewinde 6 sicherstellt, dass sich die (in den Knochen eingeschraubte) Pedikelschraube 1, 2 nicht dreht und so unbeabsichtigt weiter in den Knochen hinein- oder aus diesem herausdreht. Eine Drehung der Pedikelschraube 1, 2 um ihre Längsachse relativ zum Instrument 10 wird durch den Eingriff der Zapfen 19 in die Ausnehmungen 9 verhindert.

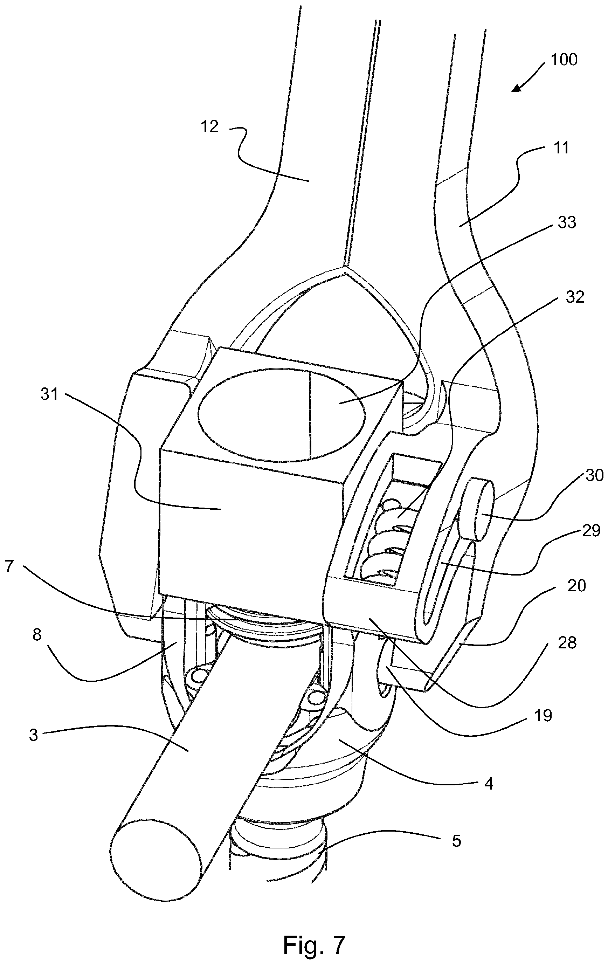

Eine zweite Ausführungsform der Erfindung in Form eines Instruments 100 ist in einer schematischen perspektivischen Darstellung in Figur 7 dargestellt. Das Instrument 100 gleicht im Wesentlichen dem in den Figuren 2 bis 6 gezeigten Instrument 10, mit folgenden Ausnahmen. Nur an dem Arbeitsende 15 der Branche 11 ist ein bogenförmiger Vorsprung 28 ausgebildet, dessen Krümmung um den Zapfen 19 verläuft. In dem Vorsprung 28 ist eine ebenfalls bogenförmige Führungsbahn 29 ausgebildet, deren Krümmung um den Zapfen 19 als Mittelpunkt verläuft. In der Führungsbahn 29 ist ein Bolzen 30 einer Führungseinheit 31 geführt. Der Radius der Krümmung der Führungsbahn 29 entspricht dem Abstand der Rotationsachse des Instruments 100 um den Kopf 4 (also der Längsachse des Zapfens 19) von der Lagerung der Führungseinrichtung 31 in der Führungsbahn 29 (also der Längsachse des Bolzens 30). Der Bolzen 30 ist in der Führungsbahn 29 auf dem Kreisbogen um den Zapfen 19 herum translatorisch verschiebbar. Da der Bolzen 30 einen runden Querschnitt aufweist, ist er außerdem um seine Längsachse drehbar. Die fest mit dem Bolzen 30 verbundene oder einteilig damit ausgeführte Führungseinheit 31 ist damit zusammen mit dem Bolzen 30 relativ zum Vorsprung 28 und zum Arbeitsende 15 translatorisch bogenförmig entsprechend der Form der Führungsbahn 29 bewegbar und um die Längsachse des Bolzens 30 drehbar. In der Führungsbahn 29 ist neben dem Bolzen 30 eine Feder 32 angeordnet, die den Bolzen 30 in die in der Figur 7 dargestellte Endlage am Ende der Führungsbahn 29 drängt.

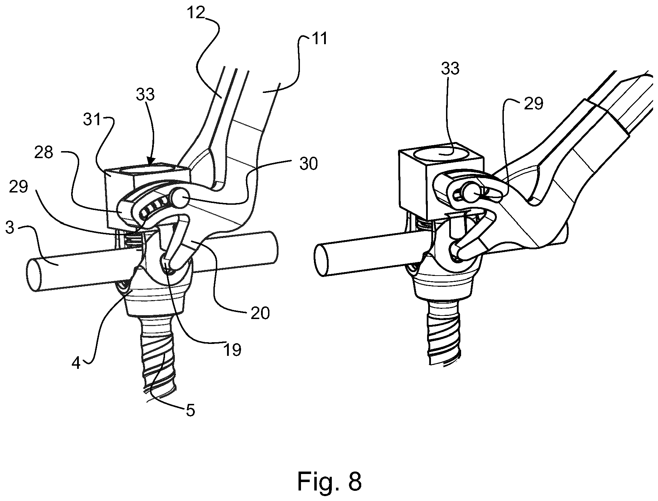

Die Führungseinheit 31 weist eine Durchgangsöffnung 33 auf, die sie in Richtung der Längsachse der Pedikelschraube 1, 2 durchdringt. Die Durchgangsöffnung 33 dient als Führung für eine nicht dargestellte Setscrew 7 bzw. für ein nicht dargestelltes Schraubwerkzeug 27. Im Gegensatz zu der Ausführungsform der Figuren 2 bis 6 wird bei der Ausführungsform der Figuren 7 und 8 eine Führung in zwei Ebenen erreicht, nämlich quer zum Stab 3 und in Richtung dessen Längsachse.

In Figur 8 ist die Verwendung dieser Ausführungsform anhand zweier unterschiedlicher Stellungen des Instruments 100 gezeigt. Figur 7 und die rechte Abbildung der Figur 8 zeigt das Instrument 100 in seiner Ausrichtung zum Ansetzen am Kopf 4 der Pedikelschraube 1, 2. In dieser Stellung ist die Führungseinheit 31 aufgrund der Wirkung der Feder 32 in die obere rechte Endlage in der Führungsbahn 29 gedrängt. Die zum Kopf 4 der Pedikelschraube 1, 2 weisende Unterseite der Führungseinheit 31 ist in dieser Position parallel zur Oberseite des Kopfs 4 ausgerichtet und liegt flach und flächig auf dieser auf. Die Zapfen 19 greifen in die seitlichen Ausnehmungen 9 der Pedikelschraube 1, 2 ein.

Nach dem Ansetzen des Instruments 100 wird dieses um die durch die Zapfen 19 und die Ausnehmungen 9 gebildete Schwenkachse geschwenkt. Da die Führungseinheit 31 flach auf der Oberseite des Kopfs 4 aufliegt, kann sie dieser Schwenkbewegung nicht folgen und verschiebt sich relativ zum Arbeitsende 15 in der Führungsbahn 29, wobei sie relativ zum Kopf 4 der Pedikelschraube in gleicher Position verbleibt. Durch die Schwenkbewegung wird der Stab 3, wie bereits im Zusammenhang mit der Ausführungsform der Figuren 2 bis 6 beschrieben, in die Stabaufnahme 8 gedrängt. Das Einschrauben der Setscrew erfolgt nun durch die Durchgangsöffnung 33 in zwei Raumrichtungen definiert geführt. Die Setscrew 7 kann dabei vor dem Ansetzen des Instruments 100 oder erst nach dem Eindrücken des Stabs 3 in die Stabaufnahme 8 in der Durchgangsöffnung eingesetzt werden. Die zweite Ausführungsform entspricht im Übrigen der ersten Ausführungsform.

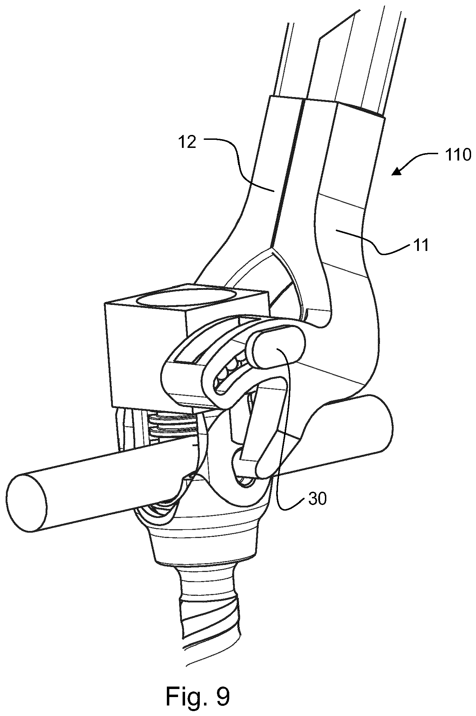

Eine dritte Ausführungsform der Erfindung in Form eines Instruments 110 ist in Figur 9 in einer schematischen perspektivischen Darstellung gezeigt. Das Instrument 110 gleicht im Wesentlichen dem Instrument 100 der Figuren 7 und 8, mit der Ausnahme, dass der Bolzen 30 keinen runden, sondern einen länglichen Querschnitt aufweist, so dass er sich zwar entlang der Führungsbahn 29 translatorisch bewegen kann, sich jedoch nicht um seine Längsachse drehen kann. Da eine Drehung der Führungseinheit 31 in der Führungsbahn 29 nicht möglich ist, ist deren korrekte Positionierung flach auf der Oberseite des Kopfs 4 der Pedikelschraube 1, 2 aufliegend stets sichergestellt.

In einer weiteren Ausführungsform, die in den Figuren nicht dargestellt ist, die jedoch den Ausführungsformen der Figuren 7 bis 9 im Wesentlichen entspricht, ist die Führungseinheit 31 hälftig geteilt. Die eine Hälfte der Führungseinheit 31 ist in der Führungsbahn 29 der Branche 11 geführt. Die andere Branche 12 weist ebenfalls einen Vorsprung 28 mit einer Führungsbahn 29 auf, in der die zweite Hälfte der Führungseinheit 31 identisch geführt ist. Die dritte und vierte Ausführungsform entspricht im Übrigen der ersten Ausführungsform.

## Patentansprüche

1. Chirurgisches Instrument (10; 100; 110) zum Manipulieren und Positionieren eines chirurgischen Stabs (3) relativ zu einem chirurgischen Implantat (1, 2), wobei
das Instrument (10; 100; 110) zangenartig mit zwei Branchen (11, 12) ausgebildet ist, die über ein Scharnier (13) drehbar miteinander gekoppelt sind;
jede der Branchen (11, 12) ein Arbeitsende (15, 17) aufweist, welche Arbeitsenden (15, 17) bestimmt und eingerichtet sind, beim Schließen des Instruments (10; 100; 110) durch Drehen der Branchen (11, 12) um das Scharnier (13) mit dem Implantat (1, 2) in Eingriff zu gelangen und das Instrument (10; 100; 110) relativ zum Implantat (1, 2) um eine Schwenkachse (21) quer zu dessen Längsachse verschwenkbar zu positionieren;
zumindest eine der Branchen (11, 12) eine Anlagestruktur (22, 23) für einen chirurgischen Stab (3) aufweist, mit der der Stab (3) bei einem Verschwenken des Instruments (10; 100; 110) um die Schwenkachse (21) relativ zu einer Stabaufnahme (8) des Implantats (1, 2) manipuliert und positioniert wird; und
an zumindest einer der Branchen (11, 12) eine Führungsstruktur (25, 26, 31, 33) ausgebildet ist, zum Führen einer nach der Positionierung des Stabs (3) in der Stabaufnahme (8) einzuschraubenden Fixierungsschraube (7) für den Stab (3) und/oder zum seitlichen Führen eines Werkzeugs (27) zum Einschrauben der nach der Positionierung des Stabs (3) in der Stabaufnahme (8) einzuschraubenden Fixierungsschraube (7) für den Stab (3),
**dadurch gekennzeichnet, dass**
an dem Arbeitsende (15, 17) zumindest einer Branche (11, 12) ein bogenförmiger Vorsprung (25) einstückig angeformt ist, wobei die Innenfläche (26) des Vorsprungs (25) eine Verlängerung der Stabaufnahme (8) und eine seitliche Führung für die Fixierungsschraube (7) und/oder das Werkzeug (27) zum Einschrauben der Fixierungsschraube (7) oder für eine dazu separat ausgebildete Führungsstruktur bildet.

2. Chirurgisches Instrument (10; 100; 110) nach Anspruch 1, **dadurch gekennzeichnet, dass** die Führungsstruktur (25, 26, 31, 33) so ausgebildet ist, dass beim Einschrauben der Fixierungsschraube (7) zunächst die Fixierungsschraube (7) und bei fortgesetztem Einschrauben der Fixierungsschraube (7) das Werkzeug (27) geführt wird.

3. Chirurgisches Instrument (10; 100; 110) nach einem der vorstehenden Ansprüche, **dadurch gekennzeichnet, dass** wenigstens ein Arbeitsende (15, 17) einen Zapfen (19) aufweist, der beim Schließen des Instruments (10; 100; 110) durch Drehen der Branchen (11, 12) um das Scharnier (13) in eine dazu vorgesehene Ausnehmung (9) in dem Implantat (1, 2) eingreift und darin relativ zum Implantat (1, 2) um die Achse (21) des Zapfens (19) drehbar gehalten ist.

4. Chirurgisches Instrument (10; 100; 110) nach einem der vorstehenden Ansprüche, **dadurch gekennzeichnet, dass** die Führungsstruktur (25, 26, 31, 33) in Form einer Führungsfläche (26, 33) ausgebildet ist, die orthogonal zur Schwenkachse (21) des Instruments (10; 100; 110) orientiert ist und unabhängig vom Schwenkwinkel des Instruments (10; 100; 110) relativ zum Implantat (1, 2) die Führung für die Fixierungsschraube (7) und/oder das Werkzeug (27) zum Einschrauben der Fixierungsschraube (7) bildet.

5. Chirurgisches Instrument (10; 100; 110) nach einem der vorstehenden Ansprüche, **dadurch gekennzeichnet, dass** die Anlagestruktur (22, 23) an einer der Branchen (11, 12) ausgebildet ist und sich bei geschlossenem Instrument (10; 100; 110) im Wesentlichen quer über den gesamten Bereich zwischen den Branchen (11, 12) erstreckt.

6. Chirurgisches Instrument (10; 100; 110) nach einem der vorstehenden Ansprüche, **dadurch gekennzeichnet, dass** die Führungsstruktur (25, 26, 31, 33) oder zumindest ein Teil davon beim Verschwenken des Instruments (10; 100; 110) mitverschwenkt wird, insbesondere um das proximale Ende des Implantats (1, 2) geschwenkt wird.

7. Chirurgisches Instrument (10; 100; 110) nach einem der vorstehenden Ansprüche, **dadurch gekennzeichnet, dass** die Führungsstruktur (25, 26, 31, 33) oder zumindest ein Teil davon über den gesamten zu erwartenden Schwenkbereich des Instruments (10; 100; 110) das Implantat (1, 2) in axialer Verlängerung der Stabaufnahme (8) seitlich einfasst.

8. Chirurgisches Instrument (10; 100; 110) nach einem der vorstehenden Ansprüche, **dadurch gekennzeichnet, dass** jede Branche (11, 12) einstückig ausgebildet ist und die Führungsstruktur (25, 26) oder zumindest ein Teil davon an zumindest einem Arbeitsende der Branche (11, 12) ausgebildet ist.

9. Chirurgisches Instrument (100; 110) nach einem der vorstehenden Ansprüche, **dadurch gekennzeichnet, dass** die Führungsstruktur eine an wenigstens einer der Branchen (11, 12) auf einem Kreisbogen um die Schwenkachse (21) geführte Führungseinheit (31) mit einer teilzylinderförmigen oder zylinderförmigen Führungsfläche für die Fixierungsschraube (7) und/oder das Werkzeug (27) zum Einschrauben der Fixierungsschraube (7) aufweist.

10. Chirurgisches Instrument (100; 110) nach Anspruch 9, **dadurch gekennzeichnet, dass** die Führungseinheit (31) einen Bolzen (30) aufweist, der in einer in der Branche (11, 12) ausgebildeten bogenförmigen Nut (29) geführt ist.

11. Chirurgisches Instrument (100; 110) nach Anspruch 10, **dadurch gekennzeichnet, dass** die Nut (29) in dem bogenförmigen Vorsprung (25) ausgebildet ist.

12. Chirurgisches Instrument (100; 110) nach Anspruch 10 oder 11, **dadurch gekennzeichnet, dass** der Bolzen (30) in der Nut (29) mittels einer Feder (32) in eine Endlage vorgespannt ist.

13. Chirurgisches Instrument (100; 110) nach einem der Ansprüche 9 bis 12, **dadurch gekennzeichnet, dass** die Führungseinheit (31) zweigeteilt ist und an jeder der Branchen (11, 12) ein Teil der Führungseinheit angeordnet ist.

14. Chirurgisches Instrument (100; 110) nach einem der Ansprüche 9 bis 13, **dadurch gekennzeichnet, dass** die Führungseinheit (31) eine zylinderförmige Durchgangsöffnung (33) aufweist, deren Innenwand die Führungsfläche für die Fixierungsschraube (7) und/oder das Werkzeug (27) zum Einschrauben der Fixierungsschraube (7) ausbildet.

## Claims

1. A surgical instrument (10; 100; 110) for manipulating and positioning a surgical rod (3) relative to a surgical implant (1, 2), wherein
the instrument (10; 100; 110) is realized as a sort of pliers comprising two legs (11, 12) which are rotatably coupled to each other by means of a hinge (13);
each of the legs (11, 12) comprises a working end (15, 17), said working ends (15, 17) being intended and arranged to come into engagement with the implant (1, 2) upon closing the instrument (10; 100; 110) by rotating the legs (11, 12) around the hinge (13) and to pivotally position the instrument (10; 100; 110) relative to the implant (1, 2) around a swivel axis (21) which is transverse to its longitudinal axis; and
at least one of the legs (11, 12) comprises a contact structure (22, 23) for a surgical rod (3), by means of which the rod (3) is manipulated and positioned relative to a rod seating (8) of the implant upon swiveling the instrument (10; 100; 110) around the swivel axis (21),
at least one of the legs (11, 12) is provided with a guide structure (25, 26, 31, 33) for guiding a fixation screw (7), to be screwed in, for the rod (3) after having positioned the rod (3) in the rod seating (8) and/or for laterally guiding a tool (27) for screwing in the fixation screw (7), to be screwed in, for the rod (3) after having positioned the rod (3) in the rod seating (8),
**characterized in that**
a curved protrusion (25) is integrally formed on the working end (15, 17) of at least one leg (11, 12), the inner surface (26) of the protrusion (25) forming a prolongation of the rod seating (8) and a lateral guide for the fixation screw (7) and/or for the tool (27) for screwing in the fixation screw (7) or for a guide structure formed separately thereto.

2. The surgical instrument (10; 100; 110) according to claim 1, **characterized in that** the guide structure (25, 26, 31, 33) is realized such that during screwing in of the fixation screw (7), at first the fixation screw (7) is guided and for continued screwing in of the fixation screw (7), the tool (27) is guided.

3. The surgical instrument (10; 100; 110) according to one of the preceding claims, **characterized in that** at least one working end (15, 17) comprises a pin (19) which upon closing the instrument (10; 100; 110) by rotating the legs (11, 12) around the hinge (13) engages a dedicated recess (9) in the implant (1, 2) and is retained therein so as to be rotatable relative to the implant (1, 2) around the axis (21) of the pin (19).

4. The surgical instrument (10; 100; 110) according to one of the preceding claims, **characterized in that** the guide structure (25, 26, 31, 33) is realized in the form of a guide surface (26, 33) which is oriented to be perpendicular to the swivel axis (21) of the instrument (10; 100; 110) and forms the guide for the fixation screw (7) and/or for the tool (27) for screwing in the fixation screw (7) independently of the swiveling angle of the instrument (10; 100; 110) relative to the implant (1, 2).

5. The surgical instrument (10; 100; 110) according to one of the preceding claims, **characterized in that** the contact structure (22, 23) is formed on one of the legs (11, 12) and extends substantially across the entire area between the legs (11, 12) if the instrument (10; 100; 110) is closed.

6. The surgical instrument (10; 100; 110) according to one of the preceding claims, **characterized in that** the guide structure (25, 26, 31, 33) or at least a part thereof is swiveled upon swiveling the instrument (10; 100; 110), and is swiveled in particular around the proximal end of the implant (1, 2).

7. The surgical instrument (10; 100; 110) according to one of the preceding claims, **characterized in that** the guide structure (25, 26, 31, 33) or at least a part thereof laterally encompasses the implant (1, 2) in axial prolongation of the rod seating (8) across the entire expectable swiveling zone of the instrument (10; 100; 110).

8. The surgical instrument (10; 100; 110) according to one of the preceding claims, **characterized in that** each leg (11, 12) is formed in one piece and the guide structure (25, 26) or at least a part thereof is arranged on at least one working end of the leg (11, 12).

9. The surgical instrument (100; 110) according to one of the preceding claims, **characterized in that** the guide structure comprises a guide unit (31) which is guided on at least one of the legs (11, 12) on a circular arc around the swivel axis (21) and comprises a partially cylindrical or cylindrical guide surface for the fixation screw (7) and/or for the tool (27) for screwing in the fixation screw (7).

10. The surgical instrument (100; 110) according to claim 9, **characterized in that** the guide unit (31) comprises a bolt (30) which is guided in a curved groove (29) formed in the leg (11, 12).

11. The surgical instrument (100; 110) according to claim 10, **characterized in that** the groove (29) is formed in the curved protrusion (25).

12. The surgical instrument (100; 110) according to claim 10 or 11, **characterized in that** the bolt (30) is prestressed in the groove (29) into an end position with the aid of a spring (32).

13. The surgical instrument (100; 110) according to one of the claims 9 to 12, **characterized in that** the guide unit (31) is two-part and each of the legs (11, 12) has one part of the guide unit arranged thereon.

14. The surgical instrument (100; 110) according to one of the claims 9 to 13, **characterized in that** the guide unit (31) comprises cylindrical through-hole (33) whose inner wall forms the guide surface for the fixation screw (7) and/or for the tool (27) for screwing in the fixation screw (7).

## Revendications

1. Instrument chirurgical (10 ; 100 ; 110) destiné à manipuler et à positionner une tige chirurgicale (3) par rapport à un implant chirurgical (1, 2),
l'instrument (10; 100; 110) étant formé en forme de pince munie de deux branches (11, 12), qui sont couplées de manière à pouvoir pivoter ensemble par l'intermédiaire d'une charnière (13) ;
chacune des branches (11, 12) présente une extrémité de travail (15, 17), lesdites extrémités de travail (15, 17) sont définies et agencées, pour venir en prise avec l'implant (1, 2) lors de la fermeture de l'instrument (10 ; 100 ; 110) par rotation des branches (11, 12) autour de la charnière (13) et positionner l'instrument (10 ; 100 ; 110) de manière à pouvoir pivoter par rapport à l'implant (1, 2) autour d'un axe de rotation (21) de manière transversale par rapport à son axe longitudinal ;
au moins l'une des branches (11, 12) présente une structure de butée (22, 23) pour une tige chirurgicale (3), avec laquelle la tige (3) lors d'une rotation de l'instrument (10 ; 100 ; 110) autour de l'axe de rotation (21) est manipulée et positionnée par rapport à une réception de tige (8) de l'implant (1, 2) ; et
une structure de guidage (25, 26, 31, 33) est formée sur au moins l'une des branches (11, 12), pour guider une vis de fixation (7) à visser après le positionnement de la tige dans la réception de tige (8) pour la tige (3) et/ou pour guider de manière latérale un outil (27) destiné à visser la vis de fixation (7) à visser après le positionnement de la tige (3) dans la réception de tige (8) pour la tige (3),
**caractérisé en ce que**
sur l'extrémité de travail (15, 17) d'au moins une branche (11, 12) une saillie (25) de forme arquée est formée d'un seul tenant, la surface intérieure (26) de la saillie (25) formant un prolongement de la réception de tige (8) et un guidage latéral pour la vis de fixation (7) et/ou l'outil (8) destiné à visser la vis de fixation (7) ou destiné à une structure de guidage conçue de manière distincte à celui-ci.

2. Instrument chirurgical (10 ; 100 ; 110) selon la revendication 1, **caractérisé en ce que** la structure de guidage (25, 26, 31, 33) est formée de sorte que lors du vissage de la vis de fixation (7) la vis de fixation (7) est guidée en premier et l'outil (27) est guidé en poursuivant le vissage de la vis de fixation (7).

3. Instrument chirurgical (10 ; 100 ; 110) selon l'une quelconque des revendications précédentes, **caractérisé en ce qu'**au moins une extrémité de travail (15, 17) présente un tenon (19), qui lors de la fermeture de l'instrument (10 ; 100 ; 110) par rotation des branches (11, 12) autour de la charnière (13) vient en prise dans un évidement (9) prévu à cet effet dans l'implant (1, 2) et est maintenu en rotation dans celui-ci par rapport à l'implant (1, 2) autour de l'axe (21) du tenon (19).

4. Instrument chirurgical (10 ; 100 ; 110) selon l'une quelconque des revendications précédentes, **caractérisé en ce que** la structure de guidage (25, 26, 31, 33) est formée sous forme d'une surface de guidage (26, 33), qui est orientée de manière orthogonale par rapport à l'axe de rotation (21) de l'instrument (10 ; 100 ; 110) et forme indépendamment de l'angle de rotation de l'instrument (10 ; 100; 110) par rapport à l'implant (1, 2) le guidage de la vis de fixation (7) et/ou de l'outil (27) pour visser la vis de fixation (7).

5. Instrument chirurgical (10 ; 100 ; 110) selon l'une quelconque des revendications précédentes, **caractérisé en ce que** la structure de butée (22, 23) est formée sur l'une des branches (11, 12) et s'étend en cas d'instrument (10 ; 100 ; 110) fermé sensiblement de manière transversale sur la totalité de la zone située entre les branches (11, 12).

6. Instrument chirurgical (10 ; 100 ; 110) selon l'une quelconque des revendications précédentes, **caractérisé en ce que** la structure de guidage (25, 26, 31, 33) ou au moins une partie de celle-ci oscille avec celle-ci lors de la rotation de l'instrument (10 ; 100 ; 110), en particulier celle-ci pivote autour de l'extrémité proximale de l'implant (1, 2).

7. Instrument chirurgical (10 ; 100 ; 110) selon l'une quelconque des revendications précédentes, **caractérisé en ce que** la structure de guidage (25, 26, 31, 33) ou au moins une partie de celle-ci borde latéralement l'implant (1, 2) dans le prolongement axial de la réception de tige (8) sur la totalité de la zone de rotation attendue.

8. Instrument chirurgical (10 ; 100 ; 110) selon l'une quelconque des revendications précédentes, **caractérisé en ce que** chaque branche (11, 12) est formée d'un seul tenant et la structure de guidage (25, 26) ou au moins une partie de celle-ci est formée sur au moins une extrémité de travail de la branche (11, 12).

9. Instrument chirurgical (100, 110) selon l'une quelconque des revendications précédentes, **caractérisé en ce que** la structure de guidage présente sur un arc de cercle autour de l'axe de rotation (21) une unité de guidage (31) guidée avec une surface de guidage de forme partiellement cylindrique ou de forme cylindrique pour la vis de fixation (7) et/ou l'outil (27) pour visser la vis de fixation (7).

10. Instrument chirurgical (100 ; 110) selon la revendication 9, **caractérisé en ce que** l'unité de guidage (31) présente un boulon (30), qui est guidé dans une rainure (29) formée dans la branche (11, 12).

11. Instrument chirurgical (100; 110) selon la revendication 10, **caractérisé en ce que** la rainure (29) est formée dans la saillie (25) de forme arquée.

12. Instrument chirurgical (100 ; 110) selon la revendication 10 ou 11, **caractérisé en ce que** le boulon (30) est précontraint dans la rainure (29) au moyen d'un ressort (32) dans une position finale.

13. Instrument chirurgical (100; 110) selon l'une quelconque des revendications 9 à 12, **caractérisé en ce que** l'unité de guidage (31) est divisée en deux et sur chacune des branches (11, 12) est disposée une partie de l'unité de guidage.

14. Instrument chirurgical (100; 110) selon l'une quelconque des revendications 9 à 13, **caractérisé en ce que** l'unité de guidage (31) présente une ouverture de passage (33) de forme cylindrique, dont la paroi interne forme la surface de guidage pour la vis de fixation (7) et/ou l'outil (27) pour visser la vis de fixation (7).
